# EUROPEAN PATENT SPECIFICATION

(11) **EP 2 367 951 B1**
(45) Date of publication and mention of the grant of the patent: **11.06.2014**
(21) Application number: 09756527.9
(22) Date of filing: 25.11.2009
(51) Int. Cl.: C12Q 1/68

(54) **COMPOSITIONS AND METHODS FOR TREATING GROWTH HORMONE DEFICIENCY**
ZUSAMMENSETZUNGEN UND VERFAHREN ZUR BEHANDLUNG VON WACHSTUMSHORMONMANGEL
Compositions et procédés pour traiter la déficience en hormone de croissance

(30) Priority: 26.11.2008 EP 08170028; 26.11.2008 US 200276 P; 09.06.2009 EP 09162320
(43) Date of publication of application: 28.09.2011
(73) Proprietor: Merck Serono S.A., 1267 Coinsins (CH)
(72) Inventor: TUEFFERD, Marianne, B-2600 Berchem (Antwerp) (BE); DAUVILLIER, Jérôme, CH-1205 Genève (CH); DELAYE, Arnaud, F-01630 Saint Genis Pouilly (FR); SCHNIEPER-SAMEC, Sonia, CH-1208 Genève (CH)
(74) Representative: Merck Serono S.A. Intellectual Property
(86) International application number: PCT/EP2009/065853
(87) International publication number: WO 2010/060935

(56) References cited:
- HONG K.-W. ET AL.: "Protein Tyrosine Phosphatase N1 Gene Variants Associated with Type 2 Diabetes Mellitus and Its Related Phenotypes in the Korean Population" GENOMICS & INFORMATICS, vol. 6, no. 3, September 2008 (2008-09), pages 99-109, XP009116607 ISSN: 1598-866X
- CHEYSSAC C. ET AL.: "Analysis of common PTPN1 gene variants in type 2 diabetes, obesity and associated phenotypes in the French population" BMC MEDICAL GENETICS, BIOMED CENTRAL, LONDON, GB, vol. 7, no. 1, 5 May 2006 (2006-05-05), pages 44-53, XP002529014 ISSN: 1471-2350
- BENTO J.L. ET AL.: "Association of protein tyrosine phosphatase 1B gene polymorphisms with type 2 diabetes" DIABETES, AMERICAN DIABETES ASSOCIATION, US, vol. 53, no. 11, 1 November 2004 (2004-11-01), pages 3007-3012, XP002369381 ISSN: 0012-1797
- BINDER G. ET AL.: "The d3-growth hormone (GH) receptor polymorphism is associated with increased responsiveness to GH in turner syndrome and short small-for-gestational-age children" JOURNAL OF CLINICAL ENDOCRINOLOGY & METABOLISM, vol. 91, no. 2, February 2006 (2006-02), pages 659-664, XP002529015 ISSN: 0021-972X
- DOS SANTOS C. ET AL.: "A common polymorphism of the growth hormone receptor is associated with increased responsiveness to growth hormone" NATURE GENETICS, NATURE PUBLISHING GROUP, NEW YORK, US, vol. 36, no. 7, 1 July 2004 (2004-07-01), pages 720-724, XP002346194 ISSN: 1061-4036
- JORGE A.A.L. ET AL.: "Growth hormone (GH) pharmacogenetics: Influence of GH receptor exon 3 retention or deletion on first-year growth response and final height in patients with severe GH deficiency" JOURNAL OF CLINICAL ENDOCRINOLOGY & METABOLISM, vol. 91, no. 3, March 2006 (2006-03), pages 1076-1080, XP002529016 ISSN: 0021-972X
- TOYOSHIMA M.T.K. ET AL.: "Exon 3-deleted genotype of growth hormone receptor (GHRd3) positively influences IGF-1 increase at generation test in children with idiopathic short stature." CLINICAL ENDOCRINOLOGY OCT 2007, vol. 67, no. 4, October 2007 (2007-10), pages 500-504, XP002529017 ISSN: 0300-0664
- BUZI F. ET AL.: "Growth hormone receptor polymorphisms." ENDOCRINE DEVELOPMENT 2007, vol. 11, 2007, pages 28-35, XP001539461 ISSN: 1421-7082
- BOER DE H. ET AL.: "MONITORING OF GROWTH HORMONE REPLACEMENT THERAPY IN ADULTS, BASED ON MEASUREMENT OF SERUM MARKERS" JOURNAL OF CLINICAL ENDOCRINOLOGY AND METABOLISM, ENDOCRINE SOCIETY, CHEVY CHASE, MD, vol. 81, no. 4, 1 January 1996 (1996-01-01), pages 1371-1377, XP002918934 ISSN: 0021-972X cited in the application
- PROCTER A.M. ET AL.: "THE MOLECULAR GENETICS OF GROWTH HORMONE DEFICIENCY" HUMAN GENETICS, SPRINGER, BERLIN, DE, vol. 103, no. 3, 1 September 1998 (1998-09-01), pages 255-272, XP000990238 ISSN: 0340-6717
- MERRIAM G.R. ET AL.: "Diagnosis and treatment of growth hormone deficiency in adults: Current perspectives" CURRENT OPINION IN ENDOCRINOLOGY AND DIABETES, PHILADELPHIA, PA, US, vol. 13, no. 4, 1 January 2006 (2006-01-01), pages 362-368, XP009116635 ISSN: 1068-3097

## Description

### FIELD OF THE INVENTION

The present disclosure relates, generally, to pharmacogenetics, more specifically to genetic markers associated with the clinical response to Growth Hormone in Growth Hormone Deficiency (GHD). The present disclosure more particularly relates to human genes, which can be used for the diagnosis and treatment of Growth Hormone Deficiency (GHD).

The disclosure further discloses specific polymorphisms or alleles of several genes that are related to GHD response to early GH treatment as well as diagnostic tools and kits based on these susceptibility alterations. Thus, the disclosure can be used in the diagnosis or detection of the presence, risk or predisposition to, as well as in the prevention and/or treatment of GHD and in predicting the response to growth hormone (GH) treatment.

### BACKGROUND OF THE INVENTION

Growth Hormone Deficiency (GHD) includes a group of different pathologies all with a failure or reduction of growth hormone (GH) secretion. GHD may occur by itself or in combination with other pituitary hormone deficiencies. It may be congenital or acquired as a result of trauma, infiltrations, tumour or radiation therapy. Despite the large number of possible aetiologies, most children have idiopathic GHD. Depending on the criteria for diagnosis, the incidence of short stature associated with severe childhood GHD has been estimated to range between 1: 4000 to 1: 10000 live children in several studies (PC Sizonenko et al., Growth Horm IGF Res 2001; 11(3):137-165).

Postnatal growth of children with GHD differs according to aetiology. Genetic deficiency of GHD causes progressive slowing of growth following normal growth in the first months of life. Growth failure is the major presenting sign of GHD in children, and lack of GH therapy in the case of severe GHD leads to very short stature in adulthood (GH Research Society, J. Clin. Endocrinol Metabol 2000; 85(11): 3990-3993).

Turner (or Ullrich-Turner) syndrome (TS) is a chromosomal abnormality characterized by the absence of the entire chromosome X or a deletion within that chromosome. TS affects one in 1,500 to 2,500 live-born females. Short stature and reduced final height are observed in 95% of girls with TS. The average difference between mean adult height of normal women and that of TS adults is of 20 cm (Park E. et al, Pediatr Res 1983; 17:1-7). Reduced final height is due to a decline in height velocity after the age of 5 or 6 years (relative to unaffected girls) and to the absence of a pubertal growth spurt (Brook CGD et al., Arch Dis Child 1974; 49:789-795) due to the lack of the normal increase in GH secretion observed during puberty. The short stature in TS is not attributable to deficient secretions of GH or insulin-like growth factor I (IGF-I) (Cuttlet L et al., J Clin Endocrinol Metab 1985; 60:1087-1092), but a decreased amplitude and frequency of GH pulses have been reported after the age of 8 years in these patients (Ross JL et al., J Pediatr 1985; 106:202-206).

Recombinant DNA-derived human growth hormone (GH) is the only drug approved specifically for treatment of childhood growth failure and short stature, such as GHD, SGA (Small for Gestational Age) and TS. Current dose regimens for childhood GH therapy are based on body weight and are derived primarily from empirical experience. Variability in individual growth response to weight-based dosing in pediatric indications has led to a search for methods to optimise dosing based on other physiologic parameters. Models for prediction of GH treatment response have thus far relied on biochemical, demographic and anthropometric measures and can account for up to ∼70% of the first-year growth in response to rGH.

However, the potential additional effects of genetic variability have not been fully explored. There is thus a need to defme a set of genetic/genomic markers associated with short term GH treatment response that could complement the previously identified auxological and biochemical parameters to increase the accuracy with which response to GH treatment could be predicted.

### SUMMARY OF THE INVENTION

According to one aspect of the invention, a method is provided for identifying the level of response upon treatment with growth hormone in an individual suffering from Growth Hormone Deficiency by use of genotyping, wherein said method comprises all features of claims 1-2.

According to another aspect of the invention, growth hormone for use in treating Growth Hormone Deficiency is claimed, wherein all features of claim 3 are comprised.

### DETAILED DESCRIPTION OF THE INVENTION

The present disclosure provides novel approaches to the detection, diagnosis and monitoring of GHD in a subject, as well as for genotyping of patients having GHD. The disclosure further provides novel approaches to the treatment of GHD in a subject, and to predicting the response to growth hormone (GH) treatment thereby enabling the adjustment of the necessary dose of GH in a patient individualized manner.

Current medications to stimulate linear growth with GH in GHD include SAIZEN®. The active ingredient of SAIZEN® is somatropin, a recombinant human growth hormone (r-hGH) produced by genetically engineered mammalian cells (mouse C127). Somatropin is a single-chain, non-glycosylated protein of 191 amino acids with two disulphide bridges.

SAIZEN® is registered in many regions in the following paediatric indications:
o growth failure in children caused by decreased or absent secretion of endogenous growth hormone
o growth failure in children due to causes other than GHD (Turner Syndrome, growth disturbance in short children born SGA)
o growth failure in prepubertal children due to chronic renal failure.

SAIZEN® is also registered in 42 countries, including 15 European countries and Switzerland, in the indication of "pronounced growth hormone deficiency" in the adult.

The term "growth hormone (GH)", as used herein, is intended to include growth hormone in particular of human origin, as obtained by isolation from biological fluids or as obtained by DNA recombinant techniques from prokaryotic or eukaryotic host cells, as well as its salts, functional derivatives, variants, analogs and active fragments.

GH is a hormone with pleiotropic effects that result from the complex mechanisms regulating its synthesis and secretion as well as from the GH downstream effects resulting in the activation or inhibition of a variety of different intracellular signaling pathways, responsible for different biological effects-of GH. At the cellular level, GH binds to one single receptor, but activates multiple responses within individual target cells. GH-responsive genes include IGF-I which is the major mediator of GH action on somatic growth, and also other proteins involved in the regulation of the metabolic effects of GH. Upon administration of exogenous GH, the effects on somatic growth are long-term, but in the short term they can be evaluated by a variety of markers in peripheral blood that reflect the onset of its biological action.

Recombinant human growth hormone can typically be administered to children in a daily dosage ranging from about 0.02 mg/kg/d of body weight up to about 0.07mg/kg/d of body weight. This dosage may be given daily or accumulated as weekly dose, or the accumulated weekly dose be split into 3 or 6 equal doses per week.

The response to GH treatment, short-term as well as long-term, displays considerable inter individual variability. This is particularly evident for the endpoint of paediatric GH administration, i.e. the growth response, which varies significantly between subjects with TS but is also pronounced between children who are affected by GHD.

This variability can be investigated at two different levels. First, at the biomarker level, by assessing the individual growth response to GH administration by means of the biological markers of GH action commonly used in the clinical management of short stature subjects, as described more in detail below. Secondly, at the genotype level, which can be investigated by identifying the genetic factors responsible for the variation of biomarkers associated to the response to GH intervention.

Growth prediction models attempt to predict the individual response to GH treatment based on either pre-treatment characteristics and/or on response after a short period of GH administration in comparison to the group response. Pre-treatment parameters used in existing prediction models for idiopathic GHD and Turner Syndrome children receiving GH therapy include auxological criteria, indices of endogenous GH secretion, biological markers of GH action such as insulin-like growth factors (IGF) and their binding proteins (IGFBP), and bone turnover markers.

Response to GH treatment is evaluated herein through changes in IGF-I levels, IGF-I being a primary biomarker of GH action. In the current settings, the IGF-I levels are evaluated between baseline, briefly before the initiation of GH treatment, and 1 month of GH treatment. Low responders show a response of low magnitude in IGF-I level change between baseline and 1 month. High responders show a response of high magnitude in IGF-I level change between baseline and after short-term treatment (e.g. 1 month).

In recent years pharmacogenomics -inclusive of pharmacogenetics, as described in the present patent application - (PGx) has come into focus of physicians. Pharmacogenetics can be viewed as the study of inter-individual variations in DNA sequence as related to drug response. In this context the genome of an individual is analyzed leading to the description of genetic markers or susceptibility alterations of significance in this regard.

According to the present disclosure, the variability of the GH response was assessed by detecting genetic determinants potentially linked with changes in IGF-I levels in GH-treated GHD and TS children (genotyping). This approach is of relevance not only in evaluating the efficacy response to GH treatment but also the treatment's safety profile and long-term consequences. It has been documented that potential side effects of GH treatment include changes in insulin insensitivity and thus the development of impaired glucose tolerance, which can be monitored and depicted by standard clinical and laboratory measures. Within this context, the identification of the genetic determinants will allow prediction of individual response to GH administration and thus stratify the patients for drug administration.

In this study, components of the IGF system, including IGF binding proteins, have been selected as primary efficacy markers or biomarkers. IGF-I is the most commonly used biochemical efficacy marker of GH exposure (De Boer H et al., J Clin Endocrinol Metab 1996; 81(4):1371-1377). Serum IGF-I levels reflect GH status in conditions involving pathological GH secretion, and are consequently low in GH deficient patients. The GH-induced induction of IGF-I is rapid and reaches its maximum within a few weeks of GH administration.

Many effects of GH are mediated indirectly through IGF-I, which is produced mainly in the liver. There are six known IGF binding proteins, of which IGFBP-3 is the most abundant in the circulation and accounts for 80% of all IGF binding. Levels of both IGF-I and IGFBP-3 are strongly dependent on GH levels; thus, in GHD both measures are low. IGF-I and IGFBP-3 are not independent of each other - they usually show a relationship, which is particularly close in children.

IGF-I levels vary to a larger extent with either endogenous or exogenous GH than IGFBP-3 levels, which are more stable over time. A low pre-treatment IGF-I or IGFBP-3 serum concentration or standard deviation score (SDS) predicts a good response during the first year of treatment in children with GHD, whereas normal levels may predict a less favourable growth response (Kristrom B et al., J Clin Endocrinol Metab 1997; 82(9):2889-2898). The early changes in IGF-I and in its binding proteins (IGFBP-3) under GH treatment have been shown to correlate with baseline levels and auxological status, endogenous GH secretion, GH dose and the initial growth velocity response. IGF-I and IGFBP3 are called herein biomarkers or biological markers.

To understand the genetic factors that underlie heritable diseases or the response to pharmacological treatment, classical genetics examines a single gene or a group of a few genes of interest in relation to the trait associated to the heritable diseases or the response to pharmacological treatment. Genomics, on the other hand, allows performance of this search for genetic determinants that result in particular phenotypic characteristics at the level of the entire genome. In the present study, the following genomic techniques were used:
Genotyping: through the identification of DNA variations, this method was used to detect genetic determinants in candidate genes that are potentially linked with GHD or different response rates to GH treatment in this disease. The search for DNA variants was performed using single nucleotide polymorphisms (SNPs) as genetic markers. A SNP is a DNA locus at which the DNA sequence of two individuals carrying distinct alleles differs by one single nucleotide.

SNPs are the most common human genetic polymorphisms and their density on the genome is very high. Nearly 1.8 million SNPs have been discovered and characterized so far and are publicly available in several major databases (www.hapmap.org, October 2004). Identification of the SNPs of interest according to this disclosure can be performed with a method developed by Affymetrix or a comparable technique (Matsuzaki H et al., Genome Research 2004; 14:414-425). An association between a genetic marker (or a set of genetic markers called a haplotype) and a disease or response to treatment (the phenotype) indicates that a disease- or response- susceptibility gene may lie in the vicinity of the marker. This association is detected as a significant difference in the frequency of a particular allele at an SNP locus (or the difference in frequency of a haplotype) between patient groups with different phenotypes. This association can be detected either considering the heterozygote and homozygote status of the alleles for a given SNP, the so-called genotypic association, or on the basis of the presence of one or the other of the allele for a given SNP, the so-called allelic association. These association analyses are carried out with non parametric statistical methods, the Krustal Wallis test for genotypic and the Mann Whitney exact test for allelic.

Once a SNP has been found to be associated to a disease or response to treatment, categorical predictive analysis is required to further determine which allele is best associated to the response to treatment, and thus could serve as a predictive marker. This categorical analysis is carried out with Fisher exact test to examine the significance of the association between two variables, the response (low or high) and the genotype, in a 2 x 2 contingency table. In a further validation of these findings, the intermediate population is integrated and the tests are rerun this time in a 2 x 3 contingency table.

Moreover, predictive genetic markers are selected based on a Fisher p value less than 5% and a specificity threshold above 80%. Genetic allele frequency in the study population must be above 10% .

Exact Odds ratio together with the associated confidence interval indicated in brackets are reported as well as the predictive positive values.

Combination of 2 individual stratification genetic markers was also considered either through the "and" term or through the "or" term, this or going along the line of Boolean logic, namely requiring that either marker or both markers are present.

Complementary categorical analyses are performed for significant markers, considering the overall population, defined by three groups: Low responders, High responders, and Intermediate group (being neither Low nor High).

The terms "trait" and "phenotype" may be used interchangeably and refer to any clinically distinguishable, detectable or otherwise measurable property of an organism such as symptoms of, or susceptibility to a disease for example. Typically the terms "trait" or "phenotype" are used to refer to symptoms of, or susceptibility to GHD or TS; or to refer to an individual's response to a drug acting against GHD or TS.

As used herein, the term "allele" refers to one of the variant forms of a biallelic or multiallelic alteration, differing from other forms in its nucleotide sequence. Typically the most frequent identified allele is designated as the major allele whereas the other allele(s) are designated as minor allele(s). Diploid organisms may be homozygous or heterozygous for an allelic form.

The term "polymorphism" as used herein refers to the occurrence of two or more alternative genomic sequences or alleles between or among different genomes or individuals. "Polymorphic" refers to the condition in which two or more variants of a specific genomic sequence can be found in a population. A "polymorphic site" is the locus at which the variation occurs. A polymorphism may comprise a substitution, deletion or insertion of one or more nucleotides. A "single nucleotide polymorphism" (SNP) is a single base pair change. Typically a single nucleotide polymorphism is the replacement of one nucleotide by another nucleotide at the polymorphic site.

As will be discussed below in more details, the alteration ("susceptibility alteration") in a gene or polypeptide according to the disclosure may be any nucleotide or amino acid alteration associated to the response to growth hormone (GH) treatment in GHD children.

An allelic marker is defmed as a marker wherein the major allele is present either homozygously or heterozygously.

A genotypic marker is defined by an association between response and at least one of the three possible genotypes, homozygous for the major allele, homozygous for the minor allele or heterozygous.

Markers are selected based on continuous genetic analyses in the whole study population separated in a GHD population.

A susceptibility may be any form of mutation(s), deletion(s), rearrangement(s) and/or insertion(s) in the coding and/or non-coding region of the gene, either isolated or in various combination(s). Mutations more specifically include point mutations. Deletions may encompass any region of one or more residues in a coding or non-coding portion of the gene. Typical deletions affect small regions, such as domains (introns) or repeated sequences or fragments of less than about 50 consecutive base pairs, although larger deletions may occur as well. Insertions may encompass the addition of one or several residues in a coding or non-coding portion of the gene. Insertions may typically comprise an addition of between 1 and 50 base pairs in the gene. Rearrangements include for instance sequence inversions. An alteration may also be an aberrant modification of the polynucleotide sequence, and may be silent (i.e., create no modification in the amino acid sequence of the protein), or may result, for instance, in amino acid substitutions, frameshift mutations, stop codons, RNA splicing, e.g. the presence of a non-wild type splicing pattern of a messenger RNA transcript, or RNA or protein instability or a non-wild type level of the polypeptide. Also, the alteration may result in the production of a polypeptide with altered function or stability, or cause a reduction or increase in protein expression levels.

Typical susceptibility alterations or genetic markers are single nucleotide polymorphisms (SNPs) as described above.

The presence of an alteration in a gene may be detected by any technique known per se to the skilled artisan, including sequencing, pyrosequencing, selective hybridisation, selective amplification and/or mass spectrometry including matrix-assisted laser desorption/ionization time-of-flight mass spectrometry (MALDI-TOF MS). In a particular embodiment, the alteration is detected by selective nucleic acid amplification using one or several specific primers. The alteration is detected by selective hybridization using one or several specific probes.

Further techniques include gel electrophoresis-based genotyping methods such as PCR coupled with restriction fragment length polymorphism (RFLP) analysis, multiplex PCR, oligonucleotide ligation assay, and minisequencing; fluorescent dye-based genotyping technologies such as oligonucleotide ligation assay, pyrosequencing, single-base extension with fluorescence detection, homogeneous solution hybridization such as TaqMan, and molecular beacon genotyping; rolling circle amplification and Invader assays as well as DNA chip-based microarray and mass spectrometry genotyping technologies.

Protein expression analysis methods are known in the art and include 2-dimensional gel-electrophoresis, mass spectrometry and antibody microarrays.

Sequencing can be carried out using techniques well known in the art, e.g. using automatic sequencers. The sequencing may be performed on the complete gene or, more preferably, on specific domains thereof, typically those known or suspected to carry deleterious mutations or other alterations.

Amplification may be performed according to various techniques known in the art, such as by polymerase chain reaction (PCR), ligase chain reaction (LCR) and strand displacement amplification (SDA). These techniques can be performed using commercially available reagents and protocols. A preferred technique is allele-specific PCR.

The term "gene" as used herein shall be construed to include any type of coding nucleic acid region, including genomic DNA (gDNA), complementary DNA (cDNA), synthetic or semi-synthetic DNA, any form of corresponding RNA (e.g., mRNA), etc., as well as non coding sequences, such as introns, 5'- or 3'-untranslated sequences or regulatory sequences (e.g., promoter or enhancer), etc. The term gene particularly includes recombinant nucleic acids, i.e., any non naturally occurring nucleic acid molecule created artificially, e.g., by assembling, cutting, ligating or amplifying sequences. A gene is typically double-stranded, although other forms may be contemplated, such as single-stranded. Genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. The term "gene" may comprise any and all splicing variants of said gene.

The term "polypeptide" designates, within the context of this disclosure, a polymer of amino acids without regard to the length of the polymer; thus, peptides, oligopeptides, and proteins are included within the definition of polypeptide. A fragment of a polypeptide designates any portion of at least 8 consecutive amino acids of a sequence of said protein, preferably of at least about 15, more preferably of at least about 20, further preferably of at least 50, 100, 250, 300 or 350 amino acids. This term also includes post-translational or post-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides variants which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring.

The term "treat" or "treating" as used herein is meant to ameliorate, alleviate symptoms, eliminate the causation of the symptoms either on a temporary or permanent basis, or to prevent or slow the appearance of symptoms of the named disorder or condition. The term "treatment" as used herein also encompasses the term "prevention of the disorder", which is, e.g., manifested by delaying the onset of the symptoms of the disorder to a medically significant extent. Treatment of the disorder is, e.g., manifested by a decrease in the symptoms associated with the disorder or an amelioration of the reoccurrence of the symptoms of the disorder.

"Response" to growth hormone treatment in an individual suffering from GHD in the sense of the present disclosure is understood to be residual disease activity upon growth hormone treatment. More specifically the residual disease activity is herein associated to changes in biomarkers as a function of GH administration to these individuals suffering from GHD.

"High responders" or "good responders" refers to those individuals who can be identified to show improved response to growth hormone treatment in comparison to the GHD population who exhibit an average response level upon growth hormone treatment. The "high response" or "good response" is exhibited by reduced residual disease activity. More specifically the residual disease activity in "high responders" or "good responders" is herein associated to changes in biomarkers as a function of GH administration to these individuals suffering from GHD.

"Low responders" or "poor responders" refers to those individuals who can be identified to show impaired response to growth hormone treatment in comparison to the GHD population who exhibit an average response level upon growth hormone treatment. The "low response" or "poor response" is exhibited by increased residual disease activity. More specifically the residual disease activity in "low responders" or "low responders" is herein associated to changes in biomarkers as a function of GH administration to these individuals suffering from GHD or TS.

The present disclosure stems from the pharmacogenomics analysis evaluating gene expression and gene variations in a group of 310 GHD patients.

In the specific examples as disclosed in the present patent application, extreme categories required for categorical genetic analyses are defined by quartiles:
- the low responders are herein represented by the first and lower quartile (designated as Q1) also designated by the lowest 25% of the data (25th percentile);
- the high responders are herein represented by the third quartile and upper quartile (designated as Q3) also designated by the highest 75% (75th percentile);
- the intermediate group is herein represented as the data from >Q1 and <Q3 also designated as the intermediary 50% of the data
in GHD. In the below, the Odds Ratio (OR) describe the difference between extremes categories (Q1 and Q3); unless otherwise specified.

The results of the studies according to the disclosure show in particular for the GHD population:
- one SNP that may be used to identify low responders to treatment with GH: rs941798 in the PTPN1 gene as a genomic marker
- two SNPs that may be used to identify high responders to treatment with GH: rs2270777 in the CDK4 gene as a genomic marker, rs970467 in the LEPR gene as an allelic marker

The present disclosure provides a method of treating GHD comprising detecting the presence of the AA genotype in the genetic SNP marker rs941798 in PTPN1 gene which is predictive of a low response based on IGF-I response to GH treatment in the GHD population. The Odds ratio (OR) is 5 [1.4 -10] in the present study and thus reflects that the odds of AA being present in low responders versus high responders to the odds of AG or GG being present in low responders versus high responders in the GHD study population. Amongst the AA carriers in the low and the high responders, 73% of them are low responders; response being as per above defined as quartiles of IGF-I response in the GHD study population.

The present disclosure is thus directed in a first embodiment to a method of identifying the level of response to treatment with growth hormone in an individual having Growth Hormone Deficiency (GHD), the method comprising the steps of:
a. obtaining a DNA sample of said individual;
b. determining whether in PTPN1- rs941798 the AA genotype is present; and
c. predicting from the result of step b the response to treatment with growth hormone.

The present disclosure provides a method of treating GHD comprising detecting the presence of the AA genotype in the genetic SNP marker rs2270777 in CDK4 which is predictive of a high response based on IGF-I response to GH treatment in the GHD population. The Odds ratio (OR) is 6.64 [1.7-25.5] in the present study and thus reflects that the odds of AA being present in high responders versus low responders to the odds of AG or GG being present in high responders versus low responders in the GHD study population. Amongst the AA carriers in the low and the high responders, 82% of them are high responders; response being as per above defined as quartiles of IGF-I response in the GHD study population.

The present disclosure is thus directed in a second embodiment to a method of identifying the level of response to treatment with growth hormone in an individual having Growth Hormone Deficiency (GHD), the method comprising the steps of:
a. obtaining a DNA sample of said individual;
b. determining whether in CDK4 rs2270777 the AA genotype is present; and
c. predicting from the result of step b the response to treatment with growth hormone.

The present disclosure provides a method of treating GHD comprising detecting the presence of the A allele in the genetic SNP marker rs970467 in LEPR which is predictive of a high response based on IGF-I response to GH treatment in the GHD population. The Odds ratio (OR) is 4.80 [1.3-22.0] in the present study and thus reflects that the odds of A being present, as AA or AG, in high responders versus low responders to the odds of GG genotype being present in high responders versus low responders in the GHD study population. Amongst the A allele carriers in the low and the high responders, 77% of them are high responders; response being as per above defined as quartiles of IGF-I response in the GHD study population.

The present disclosure is thus directed in a third embodiment to a method of identifying the level of response to treatment with growth hormone in an individual having Growth Hormone Deficiency (GHD), the method comprising the steps of:
a. obtaining a DNA sample of said individual;
b. determining whether in LEPR rs970467 A allele is present; and
c. predicting from the result of step b the response to treatment with growth hormone.

The present disclosure also provides a combination of the independent predictors described above for the GHD study population including PTPN1 (AA genotype in rs941798) and not CDK4 (GG or GA genotype in rs2270777 when the CDK4 marker on an individual basis was the AA genotype): OR of 10 [2.6-33.3] for low responders. Amongst the PTPN1 AA genotype carriers and CDK4 GG or GA genotype carriers in the low and the high responders, 86% of them are low responders; response being as per above defined as quartiles of IGF-I response in the GHD study population.

The present disclosure is thus directed in a fourth embodiment to a method of identifying the level of response to treatment with growth hormone in an individual having Growth Hormone Deficiency (GHD), the method comprising the steps of:
a. obtaining a DNA sample of said individual;
b. determining whether in PTPN1 rs941798 AA genotype and in CDK4 rs2270777 the GG or GA genotype are present; and
c. predicting from the result of step b the response to treatment with growth hormone.

The present disclosure also provides a combination of the independent predictors described above for the GHD study population including CDK4 (AA genotype in rs2270777) or LEPR (A allele in rs970467): OR of 7.90 [2.8-22.2] for high responders. Amongst the CDK4 AA genotype carriers or LEPR A allele carriers in the low and the high responders, 78% of them are high responders response being as per above defined as quartiles of IGF-I response in the GHD study population.

The present disclosure is thus directed in a fifth embodiment to a method of identifying the level of response to treatment with growth hormone in an individual having Growth Hormone Deficiency (GHD), the method comprising the steps of:
a. obtaining a DNA sample of said individual;
b. determining whether in CDK4 rs2270777 AA genotype or in LEPR rs970467 the A allele is present; and
c. predicting from the result of step b the response to treatment with growth hormone.

A, T, C and G represent adenine, thymine, cytosine and guanine, respectively.

DNA samples according to the present disclosure may be obtained by taking blood samples from an individual.

In a further embodiment the present disclosure is directed to a kit for detecting a genetic markers or a combination of genetic markers that are associated with the level of response to treatment with growth hormone, as previously stated in association to biomarker response to GH treatment and in this particular case to IGF-I response.

The kit comprises a probe or a set of oligonucleotide primers designed for identifying each of the alleles in PTPN1- rs941798 and/or in CDK4 - rs2270777 and/or in LEPR - rs970467 and/or in ARRB1 - rs2276310 and/or in CDK4 - rs2069502 and/or in BCL2 - rs4456611 and/or in SH2B2 - rs2906713 and/or in PIK3CB - rs10513055.

Probes and primers that can be used according to the disclosure preferably are fragments of sequences or hybridize to the sequences shown to be associated with 1 month IGF-I changes in response to GH treatment.

The results according to this disclosure may be applied in approaches of personalized medicine. Personalized medicine is, according to the present patent application, the use of information and data from a patient's genotype to stratify disease, select a medication, provide a therapy, or initiate a preventative measure that is particularly suited to that patient at the time of administration. In addition to genetic information, other factors, including imaging, laboratory, and clinical information about the disease process or the patient play an equally important role. It is believed that personalized medicine will make it possible in the future to give the appropriate drug, at the appropriate dose, to the appropriate patient, at the appropriate time.

Patients with a genotype predictive of a high response can be given the standard dose of GH, i.e. the dose currently used in clinial practice, which is for children a daily dosage ranging from about 0.02 mg/kg of body weight up to about 0.07 mg/kg of body weight. Alternatively these patients can be given an optimized dose. Patients with markers predictive of a low response would be given an optimized dose of GH. An optimized dose of GH to be given to a low responder may be an increased dose of GH compared to the standard dose as a dose-response relationship in terms of height velocity in the first 2 years of treatment has been demonstrated; and this in a dose range compatible with the fixed dose used to treat GHD patients in the current settings. Low responders can also be candidate patients for therapies with long acting analogues of GH with a frequency of administration which is decreased.

In a further embodiment the present disclosure is thus directed to a method for treating Growth Hormone Deficiency (GHD) in an individual in need thereof, the method comprising the steps of:
a. identifying the level of response to treatment with growth hormone according to any of the methods described above,
b. treating the individual with growth hormone.

In a preferred embodiment, the individual is identified as low responder and is treated with a dose of growth hormone that is optimized compared to the standard dose.

In one embodiment, a low responder is treated with a dose of growth hormone that is increased compared to the standard dose.

Genetic markers were identified as being associated to low or high response, the response herein described being the IGF-I change after one 1 month of GH treatment. These genetic markers can be considered either alone or in combination in the methods according to the disclosure.

In a further embodiment, the disclosure relates to the use of growth hormone in the preparation of a medicament for treating paediatric Growth Hormone Deficiency (GHD) in an individual in need thereof, wherein the individual has been identified according to any of the methods described above to be a low responder or a high responder to the treatment with growth hormone.

In a further embodiment, the present disclosure relates also to growth hormone for use in treating paediatric Growth Hormone Deficiency (GHD) in an individual in need thereof, wherein the individual has been identified according to any of the methods described above to be a low responder or a high responder to the treatment with growth hormone.

In the method of identifying, kit or method of treating according to the disclosure the growth hormone is preferably human growth hormone and more preferably recombinant human growth hormone. Particular embodiments of the disclosure refer to growth hormone as sold under the tradename SAIZEN®.

Formulations useful in a method of treating a GHD patient according to the disclosure may be a liquid pharmaceutical formulation comprising growth hormone or a reconstituted freeze-dried formulation comprising growth hormone. Preferably the formulation is stabilized by a polyol, more preferably a disaccharide and even more preferably sucrose.

In the following the present disclosure shall be illustrated by means of the following examples that are not to be construed as limiting the scope of the invention.

### EXAMPLES

### Example 1: Genotyping

### 1.1. Background

GHD and TS and the different responses to GH treatment (in terms of changes in biomarker levels after one month of treatment) in the two diseases may each be associated with a specific genetic variations in one or several genes. In the present study, the search for associations between genes containing variations, in the present disclosure SNPs, so-called susceptibility genes, and disease or response to treatment was focused on candidate genes that were selected based on the physiological role of the proteins they encode and their potential implication in the diseases, GHD and TS, or in the response to GH treatment. The list of selected candidate genes is given in Table 1.

Response to GH treatment was measured by the variation of observed biomarkers in response 1 month of treatment with GH, wherein the biomarkers were either IGF-I or IGFBP3.

**Table 1**

| **GHD OR TS RELATED GENES** | |
|---|---|
| FGF-R3 | GH-1 |
| GH-R | GHRH |
| GHRH-R | Glut4 |
| HESX-1 | IGF-1 |
| Insulin-VNTR LHX3 | LHX4 |
| POU1F1 (Pit-1) | Prop-1 |
| SHOX-1 | SHOX-2 |
| STAT-5 | |
| | |

| **GH & IGF-1 RELATED GENES** | |
|---|---|
| ALS | APS (SH2B2) |
| β Arrestin-1 (ARRB1) | GAB-1 |
| GH1 | GH-R |
| GHRH | GHRH-R |
| ID1 & ID2 | IGF-I |
| IGF-I-R | IGF-II |
| IGF-II-R | IGF-BP3 |
| IGF-BP1 | IGF-BP-2 |
| IGF-BP10 | JAK2 |
| MAP Kinase | PGDF-Rβ |
| PTP1β (PTPN1) | PI3Kinase subunits |
| p60dok | SHC1 |
| STAT-5 | SOCS-2 |
| STAT- 3 | GRB10 |
| SHPS-1 | SH2B2 |
| | |

| **INSULIN RELATED GENES** | |
|---|---|
| Adiponectin (Acrp30 or AdipoQ) | ADRβ3) |
| AKT 1 & AKT 2 | Glut4 |
| Glut1 also known as SLCA1 | GRB2 |
| Insulin (VNTR) | Insulin-R |
| IRS-1 | IRS-2 |
| IRS-4 | LEP (leptin) |
| LEP-R (Leptin-R) (Ob-R) | pp120/HA4 (CEACAM8) |
| PI3Kinase p85 | PI3Kinase p110 α and p110β (polymorphic GATA binding site) Protein-Phosphatase 1 (PP1) |
| PTP1β | PDK1 |
| PPAR γ | PPARγCo-activator1 (PGC1) |
| RAs | SHIP2 |
| SHC1 | SOS 1& 2 |
| SREBP-1c | TNFα |
| | |

| **BONE METABOLISM RELATED GENES** | |
|---|---|
| AR | Aromatase |
| ER-α | GPCRs |
| Myogenin | MyoD |
| p21 | PKCα |
| RA-R | |
| | |

| **ONCOGENES & INFLAMMATORY RELATED GENES** | |
|---|---|
| bcl-2 | c-Erb B1 |
| c-fos | c-jun |
| jun-b | c-myc |
| CDK2 CDK4 and CDK6 | Cyclin D |
| TGF-α | TGF-β |
| p53 | Ras |
| Rb | WT1 |
| | |

| **INFLAMMATION RELATED GENES** | |
|---|---|
| GATA1 | IL-4 |
| IL-6 | TNF-α |

The candidate genes selected have been previously implicated in growth, the mechanism of action of growth hormone, or in growth hormone deficiency or Turner syndrome. The purpose of the study was to investigate whether TS, GHD in association to the IGF-I response to GH treatment in these diseases is correlated with a specific DNA variant or pattern of variants. The existence of such a correlation would indicate that either the gene(s) carrying the identified variant(s) or one or more genes lying in the vicinity of the variants may be (a) susceptibility gene(s).

### 1.2. Materials and Methods

### 1.2.1. DNA samples extraction and preparation

The analysis was performed on DNA extracted from polymorphonuclear leucocytes. A total of 319 blood samples were received. Out of these 319, 3 samples were not double coded and were destroyed by the genomic laboratory. The 316 samples remaining went into the genomic analysis. Out of these 316 DNA analysed, 3 were duplicates resulting in 313 DNAs analysed corresponding to 313 patients in the Predict study. Upon transfer of the clinical data, 3 patients with DNA analysed did not have any clinical data collected. Thus 310 patients were genotyped and eligible for the association studies. Only 299, 139 TS and 160 GHD, have the baseline and one month IGF-I values required for the association described in the present patent.

DNA was extracted from 316 blood samples between November 2006 and November 2007 using a Qiagen kit (QIAamp DNA Blood Midi Kit/Lot 127140243/Ref 51185). After extraction DNA quality and quantity were controlled (QC.1 and QC.2) by measures of absorbance at wavelengths of 260 nm and 280 nm using a (Molecular Devices Spectramax Plus) spectrophotometer and electrophoresis of DNA samples on agarose gels.

QC.1: 260 nm/280 nm absorbance ratio and DNA concentration calculated from the 280 nm absorbance value.

QC.2: Electrophoresis on agarose gel.

All 316 DNA samples passed the acceptance criteria defined for QC.1: absorbance ratio between 1.7 and 2.1 and DNA concentration above 50 ng/µL

All 316 DNA samples passed the acceptance criteria defined for QC.2: for each sample, one clearly defined band visible on agarose gel after electrophoresis at a high molecular weight corresponding to non-degraded genomic DNA.

An aliquot of 3 µg of DNA from each sample was distributed into four 96 well micro-plates. Each micro-plate also contained a negative control and a reference genomic DNA (referred to as DNA 103).

The four micro-plates were assigned a name ranging from Saizen-PL1 to Saizen-PL4. The 316 samples were assigned a genotyping number ranging from 50-1657 to 50-1972.

### 1.2.2. DNA microarray technology

DNA microarray technology was used for genotyping. A microarray is an experimental tool that was developed to meet the needs of whole genome analysis to simultaneously screen a vast number of genes or gene products Due to its miniaturised format and amenability to automation, a microarray is suitable for high-throughput analysis. The technique is based on the ability of two nucleic acid molecules to selectively bind (hybridise) to one another if their sequences are complementary. A set of different nucleic acid fragments, the probe, is covalently attached at defined positions on a solid support of a few square centimetres. The genetic material to be analysed, the target, is exposed to the arrayed probe. Using the selective hybridisation property of nucleic acids, the probes are designed in such a way that they will bind only to those target molecules that are of interest in the particular investigation. Selective labelling of the bound complex and the knowledge of the identity of each probe based on its location on the array allows the identification of the target molecule.

In this experiment, the Illumina Golden gate technology protocol was used. This technology is based on 3 micron silica beads that self assemble in micro-wells on either of two substrates, fiber optic bundles or planar silica slides. When randomly assembled on one of these two substrates, the beads had a uniform spacing of ∼5.7 microns. Each bead is covered with hundreds of thousands of copies of a specific oligonucleotide that act as capture sequences.

1536 SNPs were selected from the above candidate genes and 1517 SNPs were sucessfully genotyped for all individuals and analysed in 98 candidate genes out of these1536 SNPs.

The samples were randomly distributed by the biobanking technician on four 96-well microplate. Each microplate was then processed sequentially using a different Illumina kit and Sentrix Array Matrix for each plate.

### 1.2.3. Genetics Analysis

Data analysis was performed using SAS software (SAS 9.1.3, service pack 4).

### 1.2.4. Estimation of Linkage Disequilibrium (LD) structure

The number of Linkage Disequilibrium (LD) blocks in each gene was estimated in the two disease groups by means of the "ALLELE" SAS procedure, through the JMP Genomics interface.

### 1.2.5. Statistical Testing

### • Continuous analyses

For a given phenotype (e.g. change in IGF-I SDS), genotypic data (as encoded as counts of minor allele and counts of major allele) were combined for each patient in a joined table, and major and minor allele presence indicator variables was built.

### Genotypic association

The association between the genotype (considered as a categorical variable with values 0, 1, 2) and the phenotypic quantitative variable was evaluated by Krustal Wallis test implemented by the "GLM" (general Linear Model) SAS procedure. The main output of this procedure was a table essentially giving the probability levels (p-values) for the genotype categorical effect on phenotype, for each SNP and disease group.

### Allelic association

Similarly, the association between the presence of the major allele and the biomarker quantitative variable was evaluated by Mann Whitney exact test implemented by the "GLM" SAS procedure. The same was repeated for the minor allele.

The output of these procedures was a table essentially giving the p-values for the effect on phenotype of the presence of the corresponding allele, for ach SNP and disease group.

### Selection of significantly associated SNPs and genes

A summary table was produced to join output of the association tests performed (p-value and nature of the corresponding genetic variable) together with disease type, SNP and gene names, number of SNPs tested and of LD blocks in the gene, and SNP minor allele frequency (MAF) and call rate.

For selection of significant associations, Bonferroni correction for multiple testing was applied to compute adjusted p-values based on the number of tested LD blocks in the same gene (Table 4; raw p-value).

An initial aggressive selection of genes containing SNPs eligible for association was performed by selecting observations where the MAF was greater than 0.1, so as to have a frequency of the minor allele frequency (MAF) above 10% (Table 4; MAF), the call rate greater than 0.95 (Table 4; call rate), and the initial, unadjusted p-value was lower than the nominal 0.05 significance cut-off.

The final selection of significantly associated genes was based on adjustment of the raw SNP p-values by the number of LD-blocks (Table 4; adjusted p-values), used as an estimate of the number of independent tests applied to each gene.

### • Categorical analysis: prediction

To assess a potential use in patient stratification, SNPs that were associated with the IGF-I SDS response were further analysed for their association with predefined response categories:
- Good responders, having an IGF-I response higher than the 3^{rd} quartile value of IGF-I SDS change distribution (change being the variation of IGF- I SDS level after one month of GH treatment and IGF-I SDS level evaluated briefly before treatment). In GHD, good responders were children showing a IGF-I SDS change above 1.91. In TS, good responders were children showing a IGF-I SDS change above 2.63
- Poor responders, having an IGF-I response lower than the 1^{st} quartile value of IGF-I SDS change distribution (change being the variation of IGF- I SDS level after one month of GH treatment and IGF-I SDS level evaluated briefly before treatment). In GHD, poor responders were children showing a IGF-I SDS change below 0.81. In TS, poor responders were children showing a IGF-I SDS change below 1.15

Analyses were carried out for both GHD and TS.

SNPs were selected as potential stratification markers only if they met the following criteria:
- Fisher exact test p-value below 0.05
- Specificity above 80% in one responder group
- A frequency of subjects showing the genotype per disease ≥ 10% of the high and low responders' population.

Complementary analysis was performed for those markers on the overall GH or TS population (inclusive of Low, High and Intermediate responder groups), for which Odds Ratio and frequency are presented.

### 1.3. Results

The purpose of this study was the identification of genetic markers associated to variation of observed biomarkers in response to 4 weeks of treatment with SAIZEN® (active ingredient: human recombinant growth hormone), in GHD and TS children.

### Association with IGF-I SDS response

The serum concentration of IGF-I was considered in this study as the primary surrogate biomarker of growth response. In this section, "IGF-I response" is defined as the difference between the SDS levels before and after 4 weeks of treatment with SAIZEN®.

### - Association of SNPs in candidate genes through continuous analysis

SNPs were tested for association (genotypic, major or minor allele dominance, allelic additive) in the two disease groups, yielding 9165 test p-values ( 3 tests x (1526 SNPs in GHD + 1529 SNPs in TS)). Associated SNPs through Continuous analyses are reported in Table 4.

### -Prediction analysis of SNPs through categorical analysis

Considering categories of response, an association was found for GHD children with a SNP in the PTPN1 gene, with a SNP in the CDK4 gene and with a SNP in the LEPR gene.

### • GHD children

**Table 2: Genotyping results for marker SNPs in GHD subjects**

| SNP | Gene | Marker | IGF-I SDS change < 1^{st} Quartile | | IGF-I SDS change between 1^{st} and 3^{rd} quartiles | | IGF-I SDS change > 3rd Quartile | |
|---|---|---|---|---|---|---|---|---|
| | | | Marker carriers | Marker non carriers | Marker carriers | Marker non carriers | Marker carriers | Marker non carriers |
| rs941798 | PTPN1 | AA | 19 | 21 | 19 | 61 | 7 | 33 |
| rs2270777 | CDK4 | AA | 3 | 37 | 18 | 62 | 14 | 26 |
| rs970467 | LEPR | A allele | 4 | 36 | 14 | 66 | 14 | 26 |
| rs2270777 or rs941798 | Not CDK4 and PTPN1 | rs2270777 : G allele | 18 | 22 | 13 | 67 | 3 | 37 |
| | | rs941798 : AA | | | | | | |
| rs2270777 or rs970467 | CDK4 or LEPR | rs2270777 : AA | 7 | 33 | 29 | 51 | 25 | 15 |
| | | rs970467 : A allele | | | | | | |

PTPN1, Protein-tyrosine phosphatase 1B, is the founding member of the protein tyrosine phosphatase (PTP) family, whose members are known to be signaling molecules that regulate a variety of cellular processes including cell growth, differentiation and mitotic cycle. More importantly in the context of association to the mechanism of action of GH, PTP-1B has been shown to regulate GH signaling by reducing the extent of JAK2 phosphorylation (Gu et al, MolCell Biol, 23(11):3753-62, 2003). PTP-1B is best known for being a negative regulator of insulin signaling by dephosphorylating the phosphotryosine residues of insulin receptor kinase (Ramachandran & Kennedy, Curr Top Med Chem.;3(7):749-57, 2003). Interestingly in the context of the current genetic markers described, protein tyrosine phosphatase 1B (PTP1B) has also been described to act as an inhibitor of leptin signaling in vitro (Kaszubska et al, MolCellEndo, 195(1-2):109-18, 2003).

rs941798 (A/G) is located in the chromosome 20. In GHD patients, 28% (45 out of 160) of children were homozygous for the A allele (minor allele).

Carriage of the AA genotype for rs941798 has a 42% (19 out of 45) positive predictive value in GHD children for low responders based on the one month change in IGF-I SDS (Table 2).

There is 3.25 times more low responders than non low responders in AA carriers children than in G allele carriers (OR=3.24 [1.43;7.69])

CDK4, cyclin-dependent kinase 4, is a member of the Ser/Thr protein kinase family and is a key regulator of the cell cycle G1 phase progression. In effect, CDK4's activity has been shown to be restricted to the G1-S phase during which CDK4 forms complexes with the D-type cyclins (e.g., D1 ; D2 ; D3), collectively known to control the progression of cells during the cell cycle (Brugess et al, JBiolChem, 2004, 281(15):9987-95).

In animals, CDK 4 has been shown to be indispensable for postnatal proliferation of the anterior pituitary as demonstrated in Cdk4-/-, resulting in growth retardation, diabetes and infertility. These specific endocrine phenotypes have been linked to the GH axis using mice with transgenic expression of human growth hormone-releasing hormone (GHRH) and showing pituitary hyperplasia. When a Cdk4-/- background was added on, the pituitary hyperplasia was completely abrogated (Jirawatnotai et al, J Biol Chem. 2004, 279(49):51100-6). CDK4 is known to work in tight collaboration with CDK6 which has been linked to final height (Dixon et al., Nature Genetics, 2007, 39(10):1202-1207).

rs2270777 (A/G) is located in the chromosome 12. In GHD patients, 22% (35 out of 160) of children were homozygous for the A allele (minor allele) for SNP rs2270777 (in CDK4 gene).

Carriage of the AA genotype for SNP rs2270777 has a 91% (32 out of 35) positive predictive value in GHD children for not low response based on the one month change in IGF-I SDS (Table 2).

There is 4.4 times more not low responders than low responders in AA carriers children than in G allele carriers (OR=4.4[1.3-24.1])

LEPR is the receptor for Leptin, a hormone secreted by white and brown adipocytes and considered to be central to the regulation of body composition, partly through the regulation of food intake and, although this is matter to debate, through the regulation of energy expenditure. A fall in plasma leptin levels has been associated with GH treatment in children (Tauch et al, 1999, HormRes, 50:18-21). An inverse correlation also exists between leptin and GH levels in fed and fasted women (Giustina & Veldhuis, Endocrine Reviews, 1998, 19(6): 7171-797), evidence favouring the interpretation of an in vivo regulation of growth hormone by leptin (Carro et al, Endocrinology, 1997, 139: 2203-2206).

The leptin receptor has various isoforms in multiple organs, a widesperead distribution suggesting that leptin is an important endocrine mediator. Importantly, analysis of these tissues has shown that the majority of transcripts detects are in the form of the short intracellular domain with the long form begin strongly expressed in several hypothalamic nuclei (Margetic S et al, IntJObesRelatMetabDisord. 2002;26(11):1407-33)

rs970467 (A/G) is located in the chromosome 1.

In GHD patients, 20% (32 out of 160) of children were carriers of the A allele (minor allele) for SNP rs970467 (in LEPR gene).

Carriage of the A allele for SNP rs970467 has a 43% (14 out of 32) positive predictive value in GHD children for high response based on the one month change in IGF-I SDS. There is 3 times more high responders than low or intermediary responders in AA carriers children than in A allele carriers (OR=3.02[1.22-7.5])

Combining the markers not CDK4 (rs2270777) (G allele, GG or GA,) and PTPN1 (rs941798) in GHD responders patients, 21 % (34 out of 160) of children were carriers of the G allele for SNP rs2270777 and AA genotype for SNP rs941798.

Carriage of the G allele for SNP rs2270777 and AA genotype for SNP rs941798 has a 53% (18 out of 34) positive predictive value in GHD children for low response based on the one month change in IGF-I SDS.

There is 5.25 times more low responders than non low responders in "not CDK4 and PTPN1 markers" carriers children than in non carriers (OR=5.25[2.16-12.9]).

Combining the markers CDK4 (rs2270777) or LEPR (rs970467) in GHD responders patients, 38% (61 out of 160) of children were carriers of the AA genotype for SNP rs2270777 or A allele for SNP rs970467.

Carriage of AA genotype for SNP rs2270777 or A allele for SNP rs970467 has a 88% (54 out of 61) positive predictive value in GHD children for non low responders based on the one month change in IGF-I SDS.

There is 3.8 times more non low responders than low responders in "CDK4 or LEPR markers" carriers children than in non carriers (OR= 3.8 [1.5-11]).

### • TS children

**Table 3. Genotyping results for marker SNPs in TS subjects**

| SNP | Gene | Marker | IGF-I SDS change < 1^{st} Quartile | | IGF-I SDS chage between 1^{st} and 3^{rd} quartiles | | IGF-I SDS change > 3rd Quartile | |
|---|---|---|---|---|---|---|---|---|
| | | | Marker carriers | Marker non carriers | Marker carriers | Marker non carriers | Marker carriers | Marker non carriers |
| rs2276310 | ARRB1 | A allele | 5 | 30 | 10 | 59 | 15 | 20 |
| rs4456611 | BCL2 | GG | 13 | 22 | 11 | 58 | 3 | 32 |
| rs2069502 | CDK4 | GG | 19 | 16 | 35 | 34 | 5 | 30 |
| rs2906713 | SH2B2 | C allele | 14 | 21 | 19 | 50 | 5 | 30 |
| rs10513055 | PIK3CB | C allele | 14 | 21 | 17 | 52 | 5 | 30 |
| rs2270777 | CDK4 | AA | 8 | 27 | 18 | 51 | 0 | 35 |
| rs4456611 or rs2906713 | BCL2 or SH2B2 | rs4456611: GG | 22 | 13 | 28 | 41 | 7 | 28 |
| | | rs2906713: C allele | | | | | | |
| rs4456611 or rs10513055 | BCL2 or PIK3CB | rs4456611: GG | 21 | 14 | 25 | 44 | 7 | 28 |
| | | rs1051305: C allele | | | | | | |
| rs4456611 or rs2069502 | BCL2 or CDK4 | rs4456611: GG | 28 | 7 | 41 | 28 | 7 | 28 |
| | | rs2069502: GG | | | | | | |

ARRB1, arrestin-beta 1 is a member of the arrestin/beta-arrestin protein family, cytosolic proteins that bind specific G-protein-coupled receptors (GPCR) that are phosphorylated by G-protein-coupled receptors kinases. This binding leads to a desensitization which inhibits receptor-G protein coupling and thus attenuates receptor signaling (Ferguson SS, Caron MG; 1998; Semin. Cell Dev. Biol, 1998). The end result is a dampening of cellular responses; hence the name of "arrestin" for a protein that "arrests" signal transduction. This has been extensively demonstrated with beta-adrenergic receptors, arrestin beta 1 acting as a cofactor in the beta-adrenergic receptor kinase (BARK) mediated desensitization of beta-adrenergic receptors (Von Zastrow M, Neuropsychopharmacology - 5th Generation of Progress). Arrestin beta 1 has also been shown to be crucial for ubiquitination and down-regulation of the ligand-stimulated insulin-like growth factor-1 receptor (IGF-1R) by acting as adaptor for the MDM2, an E3 ubiquite ligase that ubiquinates the IGF-1R (Girnita et al, JBiolChem, 2005, 280(26), 24412-24419).

rs2276310 (A/G) is located in the chromosome 11.

In TS patients, 21% (30 out of 139) of children were carriers of the A allele (minor allele) for SNP rs2276310 (in ARRB1 gene).

Carriage of the A allele for SNP rs2276310 has a 50% (15 out of 30) positive predictive value in TS children for high response based on the one month change in IGF-I SDS. There is 4 .34 times more high responders than low or intermediary responders in A allele carriers children than in GG carriers (OR=4.34 [1.7-11.49]).

BCL2: The Bcl-2 protein (B-cell lymphoma 2) is part of a family, the Bcl2 family, of 25 genes exerting pro-apoptotic effects for some and others anti-apoptotic effects for others. Their role in apoptosis relates to their cellular location: these proteins are integral to the outer mitochondrial membrane and govern the mitochondrial's outer membrane permeabilization (MOMP) in a feedback loop system with caspases.

Bcl-2 exerts anti-apoptotic effects, blocking the apoptotic death of some cells. IGF1 has been shown to promote resistance to apoptosis in melanoma cells through an increased expression of BCL2 (Hilmi C et al; J Invest Dermatol. 2008;128(6):1499-505). Bcl-2 is also central in the regulation of inflammatory cells and growth hormone has been shown to have a regulatory function on monocytic cells (Haeffner A et al; Eur J Immunol. 1999;29(1):334-44).

rs4456611 (A/G) is located in the chromosome 18.

In TS patients, 19% (27 out of 139) of children were carriers of the GG genotype for SNP rs4456611 (in BCL2 gene).

Carriage of the GG genotype for SNP rs4456611 has a 48% (13 out of 27) positive predictive value in TS children for low response based on the one month change in IGF-I SDS. There is 3.75 times more low responders than intermediary or high responders in GG genotype carriers children (3.75[1.4-10]).

The SH2B family to which SH2B2 belongs has been shown to promote insulin-signaling. SH2B2, previously known as ALS, was initially identified as a novel insulin receptor substrate (Moodie SA et al, J Biol Chem. 1999;274(16):11186-93), associating with phosphotyrosines situated within the activation loop of the insulin receptor via the APS Src homology 2 domain. The SH2B2 gene encodes 4 isoforms that function along the signaling pathways activated by the growth hormone (GH) receptor. More specifically, the SH2B2 proteins relate to an initiating event, the activation of JAK2 (Janus kinase 2), a GH receptor-associated tyrosine kinase. SH2B2beta and/or alpha have been shown to negatively regulate insulin signaling as well as JAK2-mediated cellular responses (Li et al, Endocrinology, 2007, 148:1615-21).

rs2906713 (C/G) is located in the chromosome 7.

In TS patients, 27% (38 out of 139) of children were carriers of the C allele for SNP rs2906713 (in SH2B2 gene).

Carriage of the carriage of the C allele for SNP rs2906713 has a 63% (24 out of 38) positive predictive value in GHD children for low response based on the one month change in IGF-I SDS. Although the exact OR are not significant in this particular case, in combination this marker proves of added value.

PIK3CB: The interaction of GH with GH receptors (GHR) on target cells promotes the association of the cellular tyrosine kinase JAK2 with the GHR. Consequential to tyrosine phosphorylation of GHR, multiple signaling cascades are activated amongst which the phosphatidylinositol 3' kinase (PI3K) cascades that have been shown to regulate the transcription of GH-responsive genes (Piwien-Pilipuk G et al, J Pediatr Endocrinol Metab. 2002;15(6):771-86 ). PI3K is composed subunits including a 110-kD catalytic subunit, which relates to PIK3CB, and an 85-kD adaptor subunit (Hu et al., 1 Mol Cell Biol. 1993;13(12):7677-88). Loss of this 110-kD subunit-kD retards cell growth, although not responsive to stimulation by insulin or other growth factors in vitro (Jia et al, Nature, 2008, 454: 776-779)

rs10513055 (C/A) is located in the chromosome 3.

In TS patients, 26% (36 out of 139) of children were carriers of the C allele for SNP rs10513055 (in PIK3CB gene).

Carriage of the carriage of the C allele for SNP rs10513055 has a 61% (22 out of 36) positive predictive value in GHD children for low response based on the one month change in IGF-I SDS. Although the exact OR are not significant in this particular case, in combination this marker proves of added value.

The CDK4 gene was functionally described above.

rs2069502 (A/G) is located in the chromosome 12.

In TS patients, 42% (59 out of 139) of children were carriers of the GG genotype for SNP rs2069502 (in CDK4 gene).

Carriage of the GG genotype for SNP rs2069502 has a 91 % (54 out of 59) positive predictive value in TS children for non high response based on the one month change in IGF-I SDS. There is 6.4 times more "non high" responders than high responders in GG carriers children than in A allele carriers (OR=6.4[2.22-22.8])

rs2270777 (A/G) is located in the chromosome 12.

In TS patients, 19% (26 out of 139) of children were carriers of the AA genotype for SNP rs2270777 (in CDK4 gene).

Carriage of the AA genotype for SNP rs2270777 has a 100% (26 out of 26) positive predictive value in TS children for non high response based on the one month change in IGF-I SDS. The OR could not be evaluated.

In TS patients, combination of markers BCL2 or SH2B2 includes carriers of the GG genotype for SNP rs4456611 (in BCL2 gene) or C allele for SNP rs2906713 (in SH2B2 gene) for 41% (57 out of 139) of children.

Carriage of the marker(s) has a 87 % (50 out of 57) positive predictive value in TS children for non high response based on the one month change in IGF-I SDS. There is 3.75 times more non high responders than high responders in SH2B2 or BCL2 marker(s) carrier children than in non markers carriers (OR=3.7[1.4-10.8])

In TS patients, combination of markers BCL2 or PIK3CB includes carriers of the GG genotype for SNP rs4456611 (in BCL2 gene) or C allele for SNP rs10513055 (in PIK3CB gene) for 38% (53 out of 139) of children.

Carriage of the marker(s) has a 87 % (46 out of 53) positive predictive value in TS children for non high response based on the one month change in IGF-I SDS. There is 3.14 times more non high responders than high responders in PIK3CB or BCL2 marker(s) carrier children than in non markers carriers (OR=3.14[1.2-9.3])

In TS patients, combination of markers BCL2 or CDK4 includes carriers of the GG genotype for SNP rs4456611 (in BCL2 gene) or GG genotype for SNP rs2069502 (in CDK4 gene) for 55% ( 76 out of 139) of children.

Carriage of the marker(s) has a 91% ( 69 out of 76 ) positive predictive value in TS children for non high response based on the one month change in IGF-I SDS. There is 7.8 times more non high responders than high responders in CDK4 or BCL2 markers carriers children than in non markers carriers (OR=7.8[2.9-23.2]).

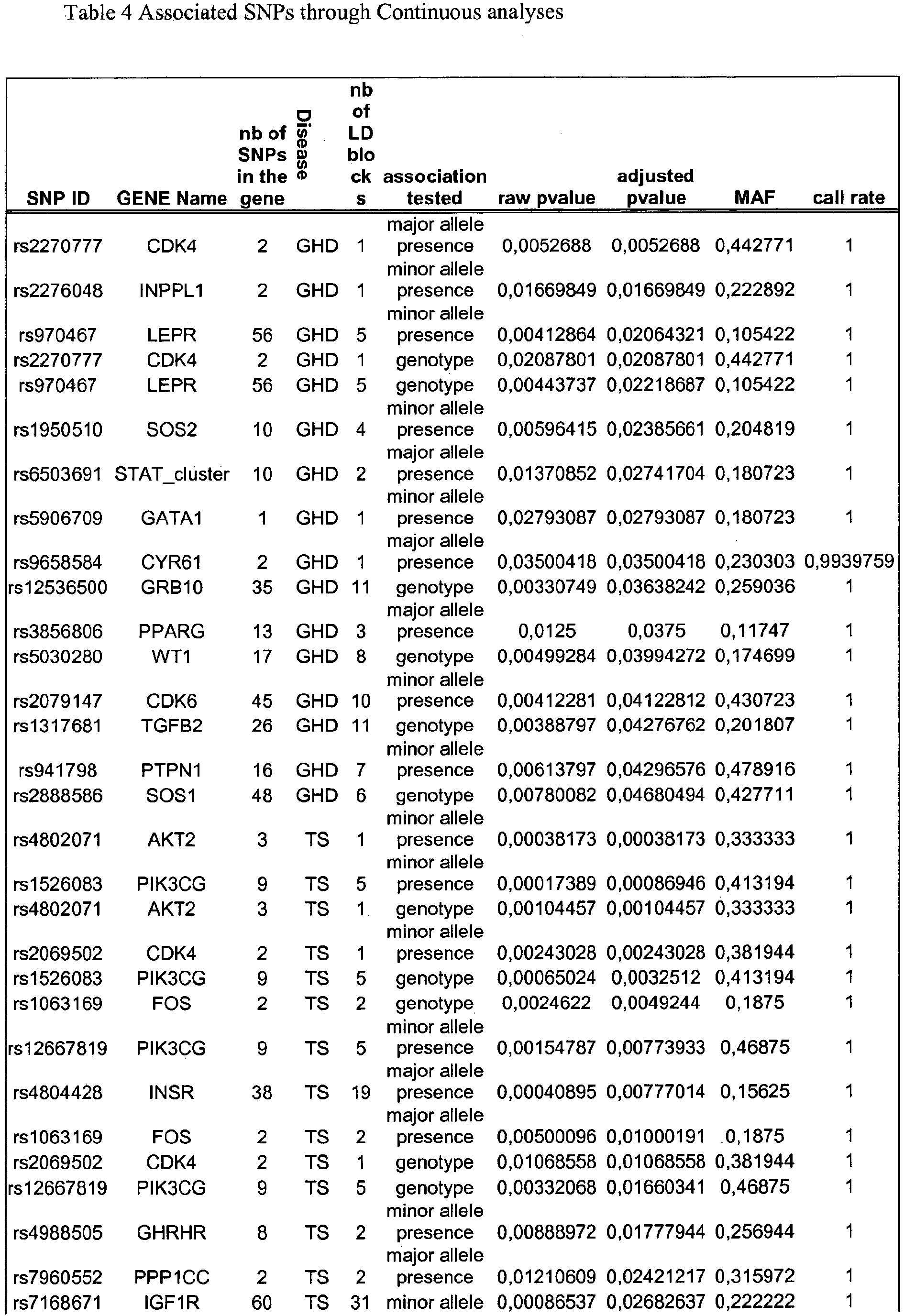

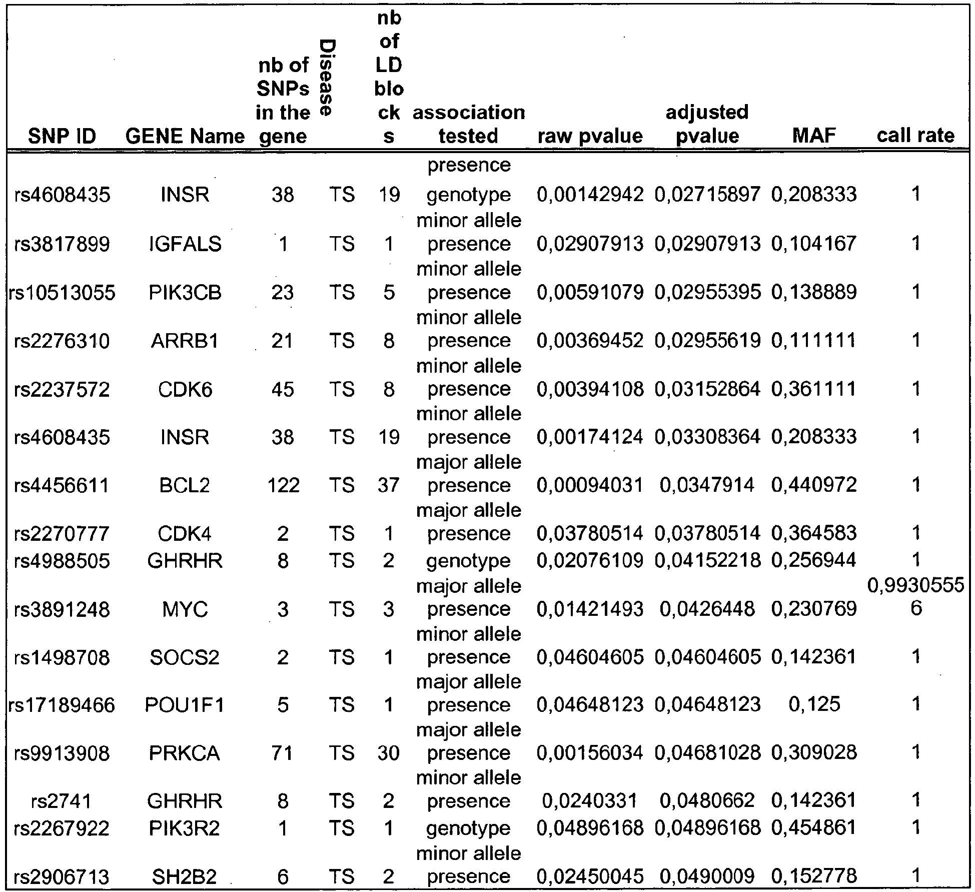

## Claims

1. A method of identifying the level of response to treatment with growth hormone in an individual having Growth Hormone Deficiency (GHD), the method comprising the steps of:
a. determining whether in PTPN1 - rs941798 the AA genotype is present;
b. predicting from the presence of the AA genotype in PTPN1 - rs941798 in a DNA sample obtained from said individual; and a low response level to treatment with growth hormone.

2. A method of identifying the level of response to treatment with growth hormone in an individual having Growth Hormone Deficiency (GHD), the method comprising the steps of:
a. determining whether in PTPN1 - rs941798 the AA genotype and in CDK4 - rs2270777 the GG or GA genotype is present; in a DNA sample obtained from said individual and
b. predicting from the presence of the AA genotype in PTPN1 - rs941798 and the presence of the GG or GA genotype in CDK4 - rs2270777 a low response level to treatment with growth hormone.

3. Growth hormone for use in treating Growth Hormone Deficiency (GHD) in an individual in need thereof wherein the individual has been identified according to the method of claim 1 or 2 to be a low responder to the treatment with growth hormone.

4. Growth hormone according to claim 3 wherein the individual is treated with a long-acting analogue of GH or with a dose of growth hormone that is increased compared to the standard dose.

5. A method according to claims 1 or 2 or a growth hormone according to claims 3 or 4 wherein the growth hormone is human growth hormone, preferably recombinant human growth hormone and more preferably Saizen®.

## Patentansprüche

1. Verfahren zum Identifizieren des Grads der Reaktion auf Behandlung mit Wachstumshormon in einem Individuum, das Wachstumshormonmangel (engl.: Growth Hormone Deficiency (GHD)) hat, wobei das Verfahren die Schritte umfasst:
a. Feststellen, ob in einer DNA-Probe, die von dem besagten Individuum gewonnen wurde, in PTPN1 - rs941798 der AA-Genotyp vorliegt und
b. Vorhersagen eines niedrigen Grads der Reaktion auf Behandlung mit Wachstumshormon aus dem Vorliegen des AA-Genotyps in PTPN1 - rs941798.

2. Verfahren zum Identifizieren des Grads der Reaktion auf Behandlung mit Wachstumshormon in einem Individuum, das Wachstumshormonmangel (GHD) hat, wobei das Verfahren die Schritte umfasst:
a. Feststellen, ob in einer DNA-Probe, die von dem besagten Individuum gewonnen wurde, in PTPN1 - rs941798 der AA-Genotyp und in CDK4 - rs2270777 der GG- oder GA-Genotyp vorliegen und
b. Vorhersagen eines niedrigen Grads der Reaktion auf Behandlung mit Wachstumshormon aus dem Vorliegen des AA-Genotyps in PTPN1 - rs941798 und aus dem Vorliegen des GG- oder GA-Genotyps in CDK4 - rs2270777.

3. Wachstumshormon zur Verwendung beim Behandeln von Wachstumshormonmangel (GHD) in einem Individuum, welches dies benötigt, wobei das Individuum gemäß dem Verfahren von Anspruch 1 oder 2 als gering auf die Behandlung mit Wachstumshormon reagierend identifiziert wurde.

4. Wachstumshormon gemäß Anspruch 3, wobei das Individuum mit einem lange wirkenden Analogon von GH oder mit einer Dosis von Wachstumshormon, die im Vergleich mit der Standarddosis gesteigert ist, behandelt wird.

5. Verfahren gemäß den Ansprüchen 1 oder 2 oder Wachstumshormon gemäß den Ansprüchen 3 oder 4, wobei es sich bei dem Wachstumshormon um humanes Wachstumshormon, bevorzugt um rekombinantes humanes Wachstumshormon und bevorzugter um Saizen^{®} handelt.

## Revendications

1. Procédé permettant de déterminer le niveau de réponse à un traitement par hormone de croissance chez un individu présentant un déficit en hormone de croissance (GHD), lequel procédé comporte les étapes suivantes :
a) déterminer si, dans un échantillon d'ADN prélevé chez ledit individu, le génotype AA est présent au niveau du SNP rs941798 du gène PTPN1 ;
b) et prédire, en se fondant sur la présence du génotype AA au niveau du SNP rs941798 du gène PTPN1, un bas niveau de réponse à un traitement par hormone de croissance.

2. Procédé permettant d'identifier le niveau de réponse à un traitement par hormone de croissance chez un individu présentant un déficit en hormone de croissance (GHD), lequel procédé comporte les étapes suivantes :
a) déterminer si, dans un échantillon d'ADN prélevé chez ledit individu, le génotype AA est présent au niveau du SNP rs941798 du gène PTPN1 et le génotype GG ou GA est présent au niveau du SNP rs2270777 du gène CDK4 ;
b) et prédire, en se fondant sur la présence du génotype AA au niveau du SNP rs941798 du gène PTPN1 et sur la présence du génotype GG ou GA au niveau du SNP rs2270777 du gène CDK4, un bas niveau de réponse à un traitement par hormone de croissance.

3. Hormone de croissance pour utilisation dans le traitement d'un déficit en hormone de croissance (GHD) chez un individu qui en a besoin, lequel individu a été identifié, par un procédé conforme à la revendication 1 ou 2, comme répondant à un bas niveau à un traitement par hormone de croissance.

4. Hormone de croissance conforme à la revendication 3, l'individu étant traité avec un analogue à longue durée d'action d'hormone de croissance ou avec une dose d'hormone de croissance accrue par rapport à la dose standard.

5. Procédé conforme à la revendication 1 ou 2, ou hormone de croissance conforme à la revendication 3 ou 4, dans lequel procédé l'hormone de croissance, ou laquelle hormone de croissance, est une hormone de croissance humaine, de préférence une hormone de croissance humaine recombinante, et mieux encore l'hormone de croissance Saizen^{®}.
